# EUROPEAN PATENT SPECIFICATION

(11) **EP 3 311 799 B1**
(45) Date of publication and mention of the grant of the patent: **11.10.2023**
(21) Application number: 17197628.5
(22) Date of filing: 20.10.2017
(51) Int. Cl.: A61K 9/00, A61K 47/34, A61K 47/60, A61K 47/69, A61L 31/14

(54) **HYDROGEL COMPOSITIONS AND DRUG DELIVERY SYSTEMS COMPRISING THE SAME**
HYDROGELZUSAMMENSETZUNGEN UND ARZNEIMITTELVERABREICHUNGSSYSTEME DAMIT
COMPOSITIONS D'HYDROGEL ET SYSTÈMES D'ADMINISTRATION DE MÉDICAMENTS

(30) Priority: 21.10.2016 US 201662411065 P; 18.10.2017 TW 106135658
(43) Date of publication of application: 25.04.2018
(73) Proprietor: Industrial Technology Research Institute, Chutung, Hsinchu 31040 (TW)
(72) Inventor: Chiang, Wen-Hsuan, 351 Toufen City, Miaoli County (TW); Lo, Yu-Wen, 220 New Taipei City (TW); Cheng, Felice, 302 Zhubei City, Hsinchu County (TW); Lu, Maggie, 310 Zhudong Township, Hsinchu County (TW); Chiu, Ya-Ling, 300 Hsinchu City (TW)
(74) Representative: Michalski Hüttermann & Partner Patentanwälte mbB

(56) References cited:
- EP-A1- 2 468 310
- EP-A1- 2 797 984
- EP-B1- 2 797 984
- US-A- 5 066 490
- US-A1- 2011 286 926
- US-A1- 2015 299 285

## Description

### TECHNICAL FIELD

The technical field relates to a novel hydrogel composition and a drug delivery system comprising the same.

### BACKGROUND

Currently, a common drug delivery carrier cannot carry high-concentration drugs. However, the use of many antibody drugs requires relatively high doses to be efficient. Therefore, the controlled-release technology of antibody drugs is facing difficult problems. In the preparation of a general carrier, the use of an organic solvent is required. However, the organic solvent often results in the inactivation of proteins. In addition, it is difficult to maintain the integrity and stability of molecular structure and biological activity of drugs during the drug-release period.

Therefore, the development of a kind of a carrier which is capable of carrying high-concentration drugs and maintaining the integrity and stability of molecular structure and biological activity of the loaded drugs during the drug-release period is desirable.

US 2011/286926 A1 describes an in situ biodegradable hydrogel drug delivery system in which the components are assembled in a manner that provides a mechanism for the timed cleavage of a particular amide bond in a covalently linked active agent or of the hydrogel structure. From EP 2 468 310 A1 compositions, methods, and kits are known for sealing applications. Compositions are prepared by combining a first cross-linkable component with a second cross-linkable component to form a porous matrix having interstices, and combining the porous matrix with a hydrogel-forming component to fill at least some of the interstices. The compositions exhibit minimal swelling properties. EP 2 797 984 A1 describes a biodegradable, biocompatible hydrogel that can be used for sealants of suppressing the leakage of blood or air during surgical operation, tissue adhesives, anti-adhesive agents and drug delivery carriers, and a method for producing the same. From US 5,066,490 crosslinking reagents for amino group-containing compounds are known, which crosslinkers can be cleaved under mildly acidic conditions. The crosslinkers can be used to crosslink biologically active substances to be delivered to the cells, wherein the crosslinker will be cleaved in the mildly acidic conditions within the cell. US 2015/299285 A1 describes Insulin analogs comprising a non-native glycosylation site sequence are provided having high potency and specificity for the insulin receptor. In one embodiment a peptide sequence of greater than 18 amino acids is used as a linking moiety to link human insulin A and B chains, or analogs or derivatives thereof, to provide high potency single chain insulin analogs. In one embodiment the linking moiety comprises one or more glycosylation sites. Also disclosed are prodrug and conjugate derivatives of the insulin analogs.

### SUMMARY

The invention is described in claim 1. Preferred embodiments of the invention are described in the sub claims. In accordance with one embodiment of the disclosure, a hydrogel composition is provided. The hydrogel composition comprises polyglutamic acid (PGA) containing maleimide groups, and polyethylene glycol (PEG) containing terminal thiol groups. Specifically, the hydrogel composition has a pH value ranging from about 4.0 to about 6.5.

In accordance with another embodiment of the disclosure, a drug delivery system as disclosed in claim 12, is provided. The drug delivery system comprises the above-mentioned hydrogel composition, and a pharmaceutically active ingredient encapsulated in the hydrogel composition.

A detailed description is given in the following embodiments with reference to the accompanying drawings.

### BRIEF DESCRIPTION OF DRAWINGS

The present disclosure can be more fully understood by reading the subsequent detailed description and examples with references made to the accompanying drawings, wherein:
FIG. 1 is a figure showing the serum concentration-time profiles according to an embodiment of the disclosure.
FIG. 2 is a figure showing the tumor-volume variation curves according to an embodiment of the disclosure.

### DETAILED DESCRIPTION

In accordance with one embodiment of the disclosure, a hydrogel composition is provided. The hydrogel composition comprises polyglutamic acid (PGA) containing maleimide groups, and polyethylene glycol (PEG) containing terminal thiol groups. Specifically, the hydrogel composition has a pH value ranging from about 4.0 to about 6.5.

In some embodiments, the polyglutamic acid (PGA) containing maleimide groups (e.g., 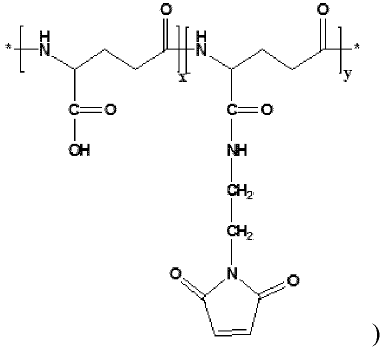 has a molecular weight ranging from about 10kDa to about 1,000kDa.

In some embodiments, the polyglutamic acid (PGA) containing maleimide groups has a grafting ratio ranging from about 5% to about 40%.

In some embodiments, the polyglutamic acid (PGA) containing maleimide groups has a concentration ranging from about 0.75wt% to about 10wt% in the hydrogel composition.

In some embodiments, the polyglutamic acid (PGA) containing maleimide groups is free of thiol groups.

In some embodiments, the polyethylene glycol (PEG) containing terminal thiol groups has a molecular weight ranging from about 2kDa to about 20kDa.

In some embodiments, the polyethylene glycol (PEG) containing terminal thiol groups has a concentration ranging from about 0.75wt% to about 10wt% in the hydrogel composition.

In some embodiments, the polyethylene glycol (PEG) containing terminal thiol groups may be 4-arm type (e.g., 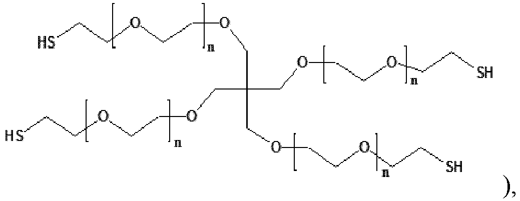 8-arm type or Y-shape.

In some embodiments, the polyethylene glycol (PEG) containing terminal thiol groups is free of maleimide groups.

In some embodiments, the polyethylene glycol (PEG) containing terminal thiol groups and the polyglutamic acid (PGA) containing maleimide groups have a molar ratio of the thiol group to the maleimide group ranging from about 0.2 to about 5.0.

In some embodiments, the polyethylene glycol (PEG) containing terminal thiol groups and the polyglutamic acid (PGA) containing maleimide groups have a molar ratio of the thiol group to the maleimide group ranging from about 1.0 to about 1.5.

In accordance with another embodiment of the disclosure, a drug delivery system is provided. The drug delivery system comprises a hydrogel composition, and a pharmaceutically active ingredient encapsulated in the hydrogel composition.

In some embodiments, the hydrogel composition may comprise polyglutamic acid (PGA) containing maleimide groups, and polyethylene glycol (PEG) containing terminal thiol groups. The hydrogel composition has a pH value ranging from about 4.0 to about 6.5.

In some embodiments, the polyglutamic acid (PGA) containing maleimide groups has a molecular weight ranging from about 10kDa to about 1,000kDa.

In some embodiments, the polyglutamic acid (PGA) containing maleimide groups has a grafting ratio ranging from about 5% to about 40%.

In some embodiments, the polyglutamic acid (PGA) containing maleimide groups has a concentration ranging from about 0.75wt% to about 10wt% in the hydrogel composition.

In some embodiments, the polyglutamic acid (PGA) containing maleimide groups is free of thiol groups.

In some embodiments, the polyethylene glycol (PEG) containing terminal thiol groups has a molecular weight ranging from about 2kDa to about 20kDa.

In some embodiments, the polyethylene glycol (PEG) containing terminal thiol groups has a concentration ranging from about 0.75wt% to about 10wt% in the hydrogel composition.

In some embodiments, the polyethylene glycol (PEG) containing terminal thiol groups may be 4-arm type, 8-arm type or Y-shape.

In some embodiments, the polyethylene glycol (PEG) containing terminal thiol groups is free of maleimide groups.

In some embodiments, the polyethylene glycol (PEG) containing terminal thiol groups and the polyglutamic acid (PGA) containing maleimide groups have a molar ratio of the thiol group to the maleimide group ranging from about 0.2 to about 5.0.

In some embodiments, the polyethylene glycol (PEG) containing terminal thiol groups and the polyglutamic acid (PGA) containing maleimide groups have a molar ratio of the thiol group to the maleimide group ranging from about 1.0 to about 1.5.

In some embodiments, the pharmaceutically active ingredient may be selected from a group consisting of peptides, proteins, growth factors, hormones, antibodies, and hydrophilic or hydrophobic small molecules.

In some embodiments, the pharmaceutically active ingredient may be selected from a group consisting of intact antibodies and antibody fragments.

In some embodiments, the pharmaceutically active ingredient may be selected from a group consisting of murine antibodies, chimeric antibodies, humanized antibodies, and human antibodies.

In some embodiments, the pharmaceutically active ingredient may be selected from a group consisting of antineoplastic agents, antipsychotics, analgesics and antibiotics.

In some embodiments, the pharmaceutically active ingredient may be further associated with a polymer, metal, charged compounds or charged particles to form a complex thereof.

In some embodiments, the polymer may comprise polyglutamic acid (PGA) or other suitable polymers such as hyaluronic acid, chitosan, alginate or dextran.

In some embodiments, the metal may comprise zinc or other suitable metals such as calcium, magnesium or iron.

In some embodiments, the complex has a size ranging from about 10nm to about 100µm.

In some embodiments, the pharmaceutically active ingredient or the complex has a concentration ranging from about 1mg/mL to about 300mg/mL.

Poly(y-glutamic acid) (y-PGA) is a high-molecular-weight polypeptide composed of γ-linked glutamic acid units and α-carboxylate side chain. γ-PGA is ideal biomaterials able to be applied to fabricate functionalized hydrogel systems because of its many advantages such us nontoxicity, hydrophilicity, biodegradability, and avoiding antigenicity or immunogenicity. In addition, the abundant carboxyl groups on the γ-PGA chains could be chemically modified, and associated with soluble antibody molecules upon electrostatic interaction.

Polyethylene glycol) (PEG) as the uncharged hydrophilic segments have been extensively employed to construct the chemically or physically cross-linked hydrogel systems due to its biocompatibility. PEG-based hydrogels have been used as cell scaffolds, adhesive medical applications, and delivery vehicles. Particularly, the ability to regulate the crosslinking density provides the flexibility and tailorability to PEG-based hydrogels for cell encapsulation and tissue growth.

In the disclosure, in order to achieve high drug loading content (beyond 150mg/mL) and long-lasting sustained drug release (over 21 days), the subcutaneously injectable and *in situ* forming hydrogel systems composed of γ-PGA and 4-arm PEG are developed. The γ-PGA is partially modified by *N*-(2-aminoethyl) maleimide upon the aminolization. The resulting maleimide-containing γ-PGA (y-PGA-MA) is mixed with 4-arm PEG-SH in aqueous solutions. Through the pH-sensitive Michael addition reaction between maleimide groups and thiol groups, the chemically cross-linked hydrogels are formed.

### Examples/Comparative Examples

### Example 1

### Preparation of the hydrogel composition (1)

First, 0.002g of polyglutamic acid (PGA) containing maleimide groups (Mw: 1,000kDa, DS (grafting ratio): 31.6%) was dissolved in 50µL of PBS to prepare a first solution (concentration: 4.0wt%). Next, 0.002g of polyethylene glycol (PEG) containing thiol groups (Mw: 5kDa, 4-arm type) was dissolved in 50µL of PBS to prepare a second solution (concentration: 4.0wt%). The first solution and the second solution were then blended at an equal volume (50µL) to prepare a hydrogel. In this preparation, the molar ratio of the thiol groups to the maleimide groups was 0.4, and the pH of the blending solution was 5.5. By visual confirmation, the gelation was successful.

### Example 2

### Preparation of the hydrogel composition (2)

First, 0.002g of polyglutamic acid (PGA) containing maleimide groups (Mw: 1,000kDa, DS (grafting ratio): 31.6%) was dissolved in 50µL of PBS to prepare a first solution (concentration: 4.0wt%). Next, 0.004g of polyethylene glycol (PEG) containing thiol groups (Mw: 5kDa, 4-arm type) was dissolved in 50µL of PBS to prepare a second solution (concentration: 8.0wt%). The first solution and the second solution were then blended at an equal volume (50µL) to prepare a hydrogel. In this preparation, the molar ratio of the thiol groups to the maleimide groups was 0.8, and the pH of the blending solution was 5.5. By visual confirmation, the gelation was successful.

### Example 3

### Preparation of the hydrogel composition (3)

First, 0.002g of polyglutamic acid (PGA) containing maleimide groups (Mw: 1,000kDa, DS (grafting ratio): 31.6%) was dissolved in 50µL of PBS to prepare a first solution (concentration: 4.0wt%). Next, 0.005g of polyethylene glycol (PEG) containing thiol groups (Mw: 5kDa, 4-arm type) was dissolved in 50µL of PBS to prepare a second solution (concentration: 10wt%). The first solution and the second solution were then blended at an equal volume (50µL) to prepare a hydrogel. In this preparation, the molar ratio of the thiol groups to the maleimide groups was 1.0, and the pH of the blending solution was 5.5. By visual confirmation, the gelation was successful.

### Example 4

### Preparation of the hydrogel composition (4)

First, 0.001g of polyglutamic acid (PGA) containing maleimide groups (Mw: 1,000kDa, DS (grafting ratio): 31.6%) was dissolved in 50µL of PBS to prepare a first solution (concentration: 2.0wt%, pH: 4.0). Next, 0.002g of polyethylene glycol (PEG) containing thiol groups (Mw: 5kDa, 4-arm type) was dissolved in 50µL of PBS to prepare a second solution (concentration: 4.0wt%, pH: 7.2). The first solution and the second solution were then blended at an equal volume (50µL) to prepare a hydrogel. In this preparation, the molar ratio of the thiol groups to the maleimide groups was 0.8, and the pH of the blending solution was 5.6. By visual confirmation, the gelation was successful and took 12 minutes.

### Example 5

### Preparation of the hydrogel composition (5)

First, 0.001g of polyglutamic acid (PGA) containing maleimide groups (Mw: 1,000kDa, DS (grafting ratio): 31.6%) was dissolved in 50µL of PBS to prepare a first solution (concentration: 2.0wt%, pH: 4.0). Next, 0.002g of polyethylene glycol (PEG) containing thiol groups (Mw: 5kDa, 4-arm type) was dissolved in 50µL of deionized water to prepare a second solution (concentration: 4.0wt%, pH: 4.4). The first solution and the second solution were then blended at an equal volume (50µL) to prepare a hydrogel. In this preparation, the molar ratio of the thiol groups to the maleimide groups was 0.8, and the pH of the blending solution was 4.3. By visual confirmation, the gelation was successful and took 20 minutes.

### Example 6

### Preparation of the hydrogel composition (6)

First, 0.001g of polyglutamic acid (PGA) containing maleimide groups (Mw: 1,000kDa, DS (grafting ratio): 31.6%) was dissolved in 50µL of deionized water to prepare a first solution (concentration: 2.0wt%, pH: 3.9). Next, 0.002g of polyethylene glycol (PEG) containing thiol groups (Mw: 5kDa, 4-arm type) was dissolved in 50µL of deionized water to prepare a second solution (concentration: 4.0wt%, pH: 4.4). The first solution and the second solution were then blended at an equal volume (50µL) to prepare a hydrogel. In this preparation, the molar ratio of the thiol groups to the maleimide groups was 0.8, and the pH of the blending solution was 4.1. By visual confirmation, the gelation was successful and took 6 hours more.

### Example 7

### Preparation of the hydrogel composition (7)

First, 0.002g of polyglutamic acid (PGA) containing maleimide groups (Mw: 1,000kDa, DS (grafting ratio): 31.6%) was dissolved in 50µL of PBS to prepare a first solution (concentration: 4.0wt%, pH: 3.9). Next, 0.002g of polyethylene glycol (PEG) containing thiol groups (Mw: 5kDa, 4-arm type) was dissolved in 50µL of deionized water to prepare a second solution (concentration: 4.0wt%, pH: 4.4). The first solution and the second solution were then blended at an equal volume (50µL) to prepare a hydrogel. In this preparation, the molar ratio of the thiol groups to the maleimide groups was 0.4, and the pH of the blending solution was 4.2. By visual confirmation, the gelation was successful and took 10 minutes.

### Example 8

### Preparation of the hydrogel composition (8)

First, 0.001g of polyglutamic acid (PGA) containing maleimide groups (Mw: 1,000kDa, DS (grafting ratio): 31.6%) was dissolved in 50µL of PBS to prepare a first solution (concentration: 2.0wt%, pH: 4.0). Next, 0.002g of polyethylene glycol (PEG) containing thiol groups (Mw: 10kDa, 4-arm type) was dissolved in 50µL of PBS to prepare a second solution (concentration: 4.0wt%, pH: 7.2). The first solution and the second solution were then blended at an equal volume (50µL) to prepare a hydrogel. In this preparation, the molar ratio of the thiol groups to the maleimide groups was 0.4, and the pH of the blending solution was 5.6. By visual confirmation, the gelation was successful and took 2-3 minutes.

### Example 9

### Preparation of the hydrogel composition (9)

First, 0.001g of polyglutamic acid (PGA) containing maleimide groups (Mw: 1,000kDa, DS (grafting ratio): 31.6%) was dissolved in 50µL of PBS to prepare a first solution (concentration: 2.0wt%, pH: 4.0). Next, 0.0015g of polyethylene glycol (PEG) containing thiol groups (Mw: 10kDa, 4-arm type) was dissolved in 50µL of PBS to prepare a second solution (concentration: 3wt%, pH: 7.2). The first solution and the second solution were then blended at an equal volume (50µL) to prepare a hydrogel. In this preparation, the molar ratio of the thiol groups to the maleimide groups was 0.3, and the pH of the blending solution was 5.6. By visual confirmation, the gelation was successful and took 6-8 minutes.

### Example 10

### Preparation of the hydrogel composition (10)

First, 0.002g of polyglutamic acid (PGA) containing maleimide groups (Mw: 200-400kDa, DS (grafting ratio): 11.5%) was dissolved in 50µL of 0.9% NaCl solution to prepare a first solution (concentration: 4.0wt%, pH: 4.2). Next, 0.004g of polyethylene glycol (PEG) containing thiol groups (Mw: 5kDa, 4-arm type) was dissolved in 50µL of 0.9% NaCl solution to prepare a second solution (concentration: 8.0wt%, pH: 5.5). The first solution and the second solution were then blended at an equal volume (50µL) to prepare a hydrogel. In this preparation, the molar ratio of the thiol groups to the maleimide groups was 2.0, and the pH of the blending solution was 4.9. By visual confirmation, the gelation was successful and took less than 5 minutes.

### Example 11

### Preparation of the hydrogel composition (11)

First, 0.002g of polyglutamic acid (PGA) containing maleimide groups (Mw: 200-400kDa, DS (grafting ratio): 11.5%) was dissolved in 50µL of 0.9% NaCl solution to prepare a first solution (concentration: 4.0wt%, pH: 4.2). Next, 0.006g of polyethylene glycol (PEG) containing thiol groups (Mw: 5kDa, 4-arm type) was dissolved in 50µL of 0.9% NaCl solution to prepare a second solution (concentration: 12.0wt%, pH: 5.5). The first solution and the second solution were then blended at an equal volume (50µL) to prepare a hydrogel. In this preparation, the molar ratio of the thiol groups to the maleimide groups was 3.0, and the pH of the blending solution was 4.9. By visual confirmation, the gelation was successful and took less than 5 minutes.

### Example 12

### Preparation of the hydrogel composition (12)

First, 0.002g of polyglutamic acid (PGA) containing maleimide groups (Mw: 200-400kDa, DS (grafting ratio): 11.5%) was dissolved in 50µL of 0.9% NaCl solution to prepare a first solution (concentration: 4.0wt%, pH: 4.2). Next, 0.01g of polyethylene glycol (PEG) containing thiol groups (Mw: 5kDa, 4-arm type) was dissolved in 50µL of 0.9% NaCl solution to prepare a second solution (concentration: 20wt%, pH: 5.5). The first solution and the second solution were then blended at an equal volume (50µL) to prepare a hydrogel. In this preparation, the molar ratio of the thiol groups to the maleimide groups was 5.0, and the pH of the blending solution was 4.9. By visual confirmation, the gelation was successful and took over one day.

### Example 13

### Preparation of the hydrogel composition (13)

First, 0.01g of polyglutamic acid (PGA) containing maleimide groups (Mw: 200-400kDa, DS (grafting ratio): 5.4%) was dissolved in 50µL of 0.9% NaCl solution to prepare a first solution (concentration: 20wt%, pH: 4.2). Next, 0.005g of polyethylene glycol (PEG) containing thiol groups (Mw: 5kDa, 4-arm type) was dissolved in 50µL of 0.9% NaCl solution to prepare a second solution (concentration: 10wt%, pH: 5.5). The first solution and the second solution were then blended at an equal volume (50µL) to prepare a hydrogel. In this preparation, the molar ratio of the thiol groups to the maleimide groups was 1.0, and the pH of the blending solution was 4.9. By visual confirmation, the gelation was successful and took 2-3 minutes.

### Example 14

### Preparation of the hydrogel composition (14)

First, 0.01g of polyglutamic acid (PGA) containing maleimide groups (Mw: 200-400kDa, DS (grafting ratio): 11.5%) was dissolved in 50µL of PBS to prepare a first solution (concentration: 20wt%, pH: 4.2). Next, 0.01g of polyethylene glycol (PEG) containing thiol groups (Mw: 5kDa, 4-arm type) was dissolved in 50µL of 0.9% NaCl solution to prepare a second solution (concentration: 20wt%, pH: 5.5). The first solution and the second solution were then blended at an equal volume (50µL) to prepare a hydrogel. In this preparation, the molar ratio of the thiol groups to the maleimide groups was 1.0, and the pH of the blending solution was 4.9. By visual confirmation, the gelation was successful and took 1-2 minutes.

### Example 15

### Preparation of the drug delivery system (1)

0.00106g of Herceptin powder was dissolved in 0.9% NaCl solution to form a drug solution (concentration: 10.6mg/mL). Next, 0.001g of polyglutamic acid (PGA) containing maleimide groups (Mw: 1,000kDa, DS (grafting ratio): 12.5%) was dissolved in 50µL of the drug solution to prepare a first solution (concentration: 2.0wt%). Next, 0.001g of polyethylene glycol (PEG) containing thiol groups (Mw: 5kDa, 4-arm type) was dissolved in 50µL of the drug solution to prepare a second solution (concentration: 2.0wt%). The first solution and the second solution were then blended at an equal volume (50µL) to prepare a hydrogel. In this preparation, the molar ratio of the thiol groups to the maleimide groups was 0.89, and the pH of the blending solution was 6.0. By visual confirmation, the gelation was successful and took 4 minutes.

### Example 16

### Preparation of the drug delivery system (2)

0.00106g of Herceptin powder was dissolved in 0.9% NaCl solution to form a drug solution (concentration: 10.6mg/mL). Next, 0.001g of polyglutamic acid (PGA) containing maleimide groups (Mw: 1,000kDa, DS (grafting ratio): 12.5%) was dissolved in 50µL of the drug solution to prepare a first solution (concentration: 2.0wt%). Next, 0.00075g of polyethylene glycol (PEG) containing thiol groups (Mw: 5kDa, 4-arm type) was dissolved in 50µL of the drug solution to prepare a second solution (concentration: 1.5wt%). The first solution and the second solution were then blended at an equal volume (50µL) to prepare a hydrogel. In this preparation, the molar ratio of the thiol groups to the maleimide groups was 0.67, and the pH of the blending solution was 6.0. By visual confirmation, the gelation was successful and took 5 minutes.

### Example 17

### Preparation of the drug delivery system (3)

0.00106g of Herceptin powder was dissolved in 0.9% NaCl solution to form a drug solution (concentration: 10.6mg/mL). Next, 0.0015g of polyglutamic acid (PGA) containing maleimide groups (Mw: 1,000kDa, DS (grafting ratio): 5.3%) was dissolved in 50µL of the drug solution to prepare a first solution (concentration: 3.0wt%). Next, 0.001g of polyethylene glycol (PEG) containing thiol groups (Mw: 5kDa, 4-arm type) was dissolved in 50µL of the drug solution to prepare a second solution (concentration: 2.0wt%). The first solution and the second solution were then blended at an equal volume (50µL) to prepare a hydrogel. In this preparation, the molar ratio of the thiol groups to the maleimide groups was 1.33, and the pH of the blending solution was 6.0. By visual confirmation, the gelation was successful and took 5 minutes.

### Example 18

### Preparation of the drug delivery system (4)

0.00106g of Herceptin powder was dissolved in 0.9% NaCl solution to form a drug solution (concentration: 10.6mg/mL). Next, 0.0015g of polyglutamic acid (PGA) containing maleimide groups (Mw: 1,000kDa, DS (grafting ratio): 5.3%) was dissolved in 50µL of the drug solution to prepare a first solution (concentration: 3.0wt%). Next, 0.00075g of polyethylene glycol (PEG) containing thiol groups (Mw: 5kDa, 4-arm type) was dissolved in 50µL of the drug solution to prepare a second solution (concentration: 1.5wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 6 minutes.

### Example 19

### Preparation of the drug delivery system (5)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 145.5mg/mL. Next, 0.0015g of polyglutamic acid (PGA) containing maleimide groups (Mw: 1,000kDa, DS (grafting ratio): 5.3%) was dissolved in 50µL of the drug solution to prepare a first solution (concentration: 3.0wt%). Next, 0.001g of polyethylene glycol (PEG) containing thiol groups (Mw: 5kDa, 4-arm type) was dissolved in 50µL of the drug solution to prepare a second solution (concentration: 2.0wt%). The first solution and the second solution were then blended at an equal volume (50µL) to prepare a hydrogel. In this preparation, the molar ratio of the thiol groups to the maleimide groups was 1.33, and the pH of the blending solution was 6.0. By visual confirmation, the gelation was successful and took 1 minute.

### Example 20

### Preparation of the drug delivery system (6)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 145.5mg/mL. Next, 0.0015g of polyglutamic acid (PGA) containing maleimide groups (Mw: 1,000kDa, DS (grafting ratio): 5.3%) was dissolved in 50µL of the drug solution to prepare a first solution (concentration: 3.0wt%). Next, 0.00075g of polyethylene glycol (PEG) containing thiol groups (Mw: 5kDa, 4-arm type) was dissolved in 50µL of the drug solution to prepare a second solution (concentration: 1.5wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 1 minute.

### Example 21

### Preparation of the drug delivery system (7)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 145.5mg/mL. Next, 0.00075g of polyglutamic acid (PGA) containing maleimide groups (Mw: 1,000kDa, DS (grafting ratio): 12.5%) was dissolved in 50µL of the drug solution to prepare a first solution (concentration: 1.5wt%). Next, 0.0006g of polyethylene glycol (PEG) containing thiol groups (Mw: 5kDa, 4-arm type) was dissolved in 50µL of the drug solution to prepare a second solution (concentration: 1.2wt%). The first solution and the second solution were then blended at an equal volume (50µL) to prepare a hydrogel. In this preparation, the molar ratio of the thiol groups to the maleimide groups was 0.71, and the pH of the blending solution was 6.0. By visual confirmation, the gelation was successful and took 1 minute.

### Example 22

### Preparation of the drug delivery system (8)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 128.6mg/mL. Next, 0.00075g of polyglutamic acid (PGA) containing maleimide groups (Mw: 200-400kDa, DS (grafting ratio): 11.3%) was dissolved in 50µL of the drug solution to prepare a first solution (concentration: 1.5wt%). Next, 0.00075g of polyethylene glycol (PEG) containing thiol groups (Mw: 5kDa, 4-arm type) was dissolved in 50µL of the drug solution to prepare a second solution (concentration: 1.5wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 20 minutes.

### Example 23

### Preparation of the drug delivery system (9)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 128.6mg/mL. Next, 0.001g of polyglutamic acid (PGA) containing maleimide groups (Mw: 200-400kDa, DS (grafting ratio): 11.3%) was dissolved in 50µL of the drug solution to prepare a first solution (concentration: 2.0wt%). Next, 0.001g of polyethylene glycol (PEG) containing thiol groups (Mw: 5kDa, 4-arm type) was dissolved in 50µL of the drug solution to prepare a second solution (concentration: 2.0wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 10 minutes.

### Example 24

### Preparation of the drug delivery system (10)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 128.6mg/mL. Next, 0.0015g of polyglutamic acid (PGA) containing maleimide groups (Mw: 200-400kDa, DS (grafting ratio): 11.3%) was dissolved in 50µL of the drug solution to prepare a first solution (concentration: 3.0wt%). Next, 0.0015g of polyethylene glycol (PEG) containing thiol groups (Mw: 5kDa, 4-arm type) was dissolved in 50µL of the drug solution to prepare a second solution (concentration: 3.0wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 4 minutes.

### Example 25

### Preparation of the drug delivery system (11)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 205mg/mL. Next, 0.00075g of polyglutamic acid (PGA) containing maleimide groups (Mw: 200-400kDa, DS (grafting ratio): 11.3%) was dissolved in 50µL of the drug solution to prepare a first solution (concentration: 1.5wt%). Next, 0.00075g of polyethylene glycol (PEG) containing thiol groups (Mw: 5kDa, 4-arm type) was dissolved in 50µL of the drug solution to prepare a second solution (concentration: 1.5wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 15-17 minutes.

### Example 26

### Preparation of the drug delivery system (12)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 205mg/mL. Next, 0.001g of polyglutamic acid (PGA) containing maleimide groups (Mw: 200-400kDa, DS (grafting ratio): 11.3%) was dissolved in 50µL of the drug solution to prepare a first solution (concentration: 2.0wt%). Next, 0.001g of polyethylene glycol (PEG) containing thiol groups (Mw: 5kDa, 4-arm type) was dissolved in 50µL of the drug solution to prepare a second solution (concentration: 2.0wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 5-6 minutes.

### Example 27

### Preparation of the drug delivery system (13)

0.1g of Herceptin powder was dissolved in deionized water and then dialyzed (MWCO: 10,000) against sodium chloride aqueous solution (0.9%) at 4°C for 24 hours to form a drug solution. Next, the drug solution was centrifuged (4,000g, 4°C) and concentrated to 205mg/mL. Next, 0.0015g of polyglutamic acid (PGA) containing maleimide groups (Mw: 200-400kDa, DS (grafting ratio): 11.3%) was dissolved in 50µL of the drug solution to prepare a first solution (concentration: 3.0wt%). Next, 0.0015g of polyethylene glycol (PEG) containing thiol groups (Mw: 5kDa, 4-arm type) was dissolved in 50µL of the drug solution to prepare a second solution (concentration: 3.0wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 2-3 minutes.

### Example 28

### Preparation of the drug delivery system (14)

0.1g of Herceptin powder was dissolved in deionized water to form a drug solution (concentration: 190mg/mL). Next, 0.001g of polyglutamic acid (PGA) containing maleimide groups (Mw: 200-400kDa, DS (grafting ratio): 11.3%) was dissolved in 50µL of the drug solution to prepare a first solution (concentration: 2.0wt%). Next, 0.001g of polyethylene glycol (PEG) containing thiol groups (Mw: 5kDa, 4-arm type) was dissolved in 50µL of the drug solution to prepare a second solution (concentration: 2.0wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took 3-5 minutes.

### Example 29

### Preparation of the drug delivery system (15)

0.1g of Herceptin powder was dissolved in deionized water to form a drug solution (concentration: 160mg/mL). Next, 0.00075g of polyglutamic acid (PGA) containing maleimide groups (Mw: 200-400kDa, DS (grafting ratio): 19.8%) was dissolved in 50µL of the drug solution to prepare a first solution (concentration: 1.5wt%). Next, 0.0015g of polyethylene glycol (PEG) containing thiol groups (Mw: 5kDa, 4-arm type) was dissolved in 50µL of the drug solution to prepare a second solution (concentration: 3.0wt%). The first solution and the second solution were then blended at an equal volume (50µL) to prepare a hydrogel. In this preparation, the molar ratio of the thiol groups to the maleimide groups was 1.0, and the pH of the blending solution was 6.0. By visual confirmation, the gelation was successful and took less than 2 minutes.

### Example 30

### Preparation of the drug delivery system (16)

0.01848g of Herceptin powder in 50uL MOPS buffer and 0.001g of polyglutamic acid (PGA) containing maleimide groups (Mw: 200-400kDa, DS (grafting ratio): 11.3%) in 50uL MOPS buffer were blended to form a complex solution and centrifuged to remove the 30uL supernatant. Next, 0.001g of polyethylene glycol (PEG) containing thiol groups (Mw: 5kDa, 4-arm type) was dissolved in 30µL of 0.9% NaCl solution and blended with the complex solution to prepare a hydrogel (final drug concentration: 184.8mg/mL). In this preparation, the molar ratio of the thiol groups to the maleimide groups was 1.0, and the pH of the blending solution was 6.0. By visual confirmation, the gelation was successful and took 5 minutes.

### Example 31

### Preparation of the drug delivery system (17)

A Herceptin/Zn complex solution having concentration of 128.0mg/mL was prepared using 0.9% NaCl solution as a buffer solution. A proper amount of polyglutamic acid (PGA) containing maleimide groups (Mw: 1,000kDa, DS (grafting ratio): 12.5%) and polyethylene glycol (PEG) containing thiol groups (Mw: 5kDa, 4-arm type) were dissolved in the Herceptin/Zn complex solution to prepare Herceptin hydrogels with PGA concentration of 1.0wt% and PEG concentration of 0.75wt% (the molar ratio of the thiol groups to the maleimide groups was 0.7) under an acidic condition with a pH value of 5.0 to prepare a hydrogel. The gelation time was more than 30 minutes.

### Example 32

### Preparation of the drug delivery system (18)

A Herceptin/Zn complex solution having concentration of 208.0mg/mL was prepared using 0.5M histidine solution as a buffer solution. A proper amount of polyglutamic acid (PGA) containing maleimide groups (Mw: 1,000kDa, DS (grafting ratio): 12.5%) and polyethylene glycol (PEG) containing thiol groups (Mw: 5kDa, 4-arm type) were dissolved in the Herceptin/Zn complex solution to prepare Herceptin hydrogels with PGA concentration of 1.3wt% and PEG concentration of 0.8wt% (the molar ratio of the thiol groups to the maleimide groups was 0.6) under an acidic condition with a pH value of 4.3 to prepare a hydrogel. The gelation time was 5-10 minutes.

### Example 33

### Preparation of the drug delivery system (19)

0.004g of Doxorubicin powder was dissolved in deionized water to form a drug solution (concentration: 4.0mg/mL). Next, 0.0015g of polyglutamic acid (PGA) containing maleimide groups (Mw: 200-400kDa, DS (grafting ratio): 11.5%) was dissolved in 50µL of the drug solution to prepare a first solution (concentration: 3.0wt%). Next, 0.0015g of polyethylene glycol (PEG) containing thiol groups (Mw: 5kDa, 4-arm type) was dissolved in 50µL of the drug solution to prepare a second solution (concentration: 3.0wt%). The first solution and the second solution were then blended at an equal volume (50µL) to prepare a hydrogel. In this preparation, the molar ratio of the thiol groups to the maleimide groups was 1.0, and the pH of the blending solution was 4.5. By visual confirmation, the gelation was successful and took 20-30 minutes.

### Comparative Example 1

### Preparation of the drug delivery system

0.002g of Doxorubicin powder was dissolved in deionized water to form a drug solution (concentration: 2.0mg/mL). Next, 0.001g of polyglutamic acid (PGA) containing maleimide groups (Mw: 200-400kDa, DS (grafting ratio): 11.5%) was dissolved in 50µL of the drug solution to prepare a first solution (concentration: 2.0wt%). Next, 0.006g of polyethylene glycol (PEG) containing thiol groups (Mw: 5kDa, 4-arm type) was dissolved in 50µL of the drug solution to prepare a second solution (concentration: 12wt%). The first solution and the second solution were then blended at an equal volume (50µL) to prepare a hydrogel. In this preparation, the molar ratio of the thiol groups to the maleimide groups was 6.0, and the pH of the blending solution was 5.0. By visual confirmation, the gelation is failed.

### Example 34

### Effect of pH on preparation of the hydrogels

Various solutions of polyglutamic acid (PGA) containing maleimide groups (Mw: 200-400kDa, DS (grafting ratio): 11.5 %) having a concentration of 1.5wt% were prepared using various buffer solutions. Various solutions of polyethylene glycol (PEG) containing thiol groups (Mw: 5kDa, 4-arm type) having a concentration of 1.5wt% were prepared using various buffer solutions. The solution of polyglutamic acid (PGA) containing maleimide groups and the solution of polyethylene glycol (PEG) containing thiol groups were mixed under various pH environments to prepare hydrogels. The gelation processes were observed. The results, such as gelation time, homogeneity and hydrogel stiffness, are shown on Table 1.

**Table 1**

| 1.5wt% PGA-MA (Mw: 200-400kDa, DS (grafting ratio): 11.5%) solution | | | | | | |
|---|---|---|---|---|---|---|
| 1.5wt% PEG-SH (Mw: 5kDa, 4-arm type) solution | | | | | | |
| Buffer solution | 0.05M carbonate buffer (pH9.6) | 0.5M PB buffer (pH7.4) | 10xPBS (pH7.8) or 0.05M PB buffer (pH7.4) | 2xPBS (pH7.8) | 1xPBS (pH7.8) | 0.1M histidine buffer (pH4.0) |
| pH value (mixture) | 8.6 | 7.2 | **6.5** | **5.0** | **4.5** | **4.2** |
| Gelation time | immediately | immediately | -30sec | ~5min | ~13min | ~20min |
| Homogeneity | very poor | poor | good | very good | very good | very good |
| Hydrogel stiffness | good | week | good | good | good | good |

In this example, under acidic pH environments (i.e. pH=4.2, 4.5, 5.0, and 6.5), appropriate gelation time and homogeneity of the mixture are obtained during the preparation. In addition, the formed hydrogel in such conditions possesses preferable stiffness.

### Comparative Example 2

### Effect of alkaline pH on encapsulation and dissolution of the drug-loaded hydrogels

A Herceptin solution having a concentration of 50.0mg/mL with a pH value of 7.8 was prepared. A proper amount of polyglutamic acid (PGA) containing maleimide groups (Mw: 1,000kDa, DS (grafting ratio): 31.6%) was dissolved in the Herceptin solution to prepare a first solution having a concentration of 2.0wt%. A proper amount of polyethylene glycol (PEG) containing thiol groups (Mw: 5kDa, 4-arm type) was dissolved in the Herceptin solution to prepare a second solution having a concentration of 4.0wt%. The first solution and the second solution were mixed (the molar ratio of the thiol groups to the maleimide groups was 0.72) under a pH value of 7.8 (an alkaline condition) to prepare a hydrogel (GAEG01). The gelation time was less than 2 minutes.

Next, a proper amount of polyglutamic acid (PGA) containing maleimide groups (Mw: 1,000kDa, DS (grafting ratio): 31.6%) was dissolved in the Herceptin solution to prepare a first solution having a concentration of 2.0wt%. A proper amount of polyethylene glycol (PEG) containing thiol groups (Mw: 10kDa, 4-arm type) was dissolved in the Herceptin solution to prepare a second solution having a concentration of 4.0wt%. The first solution and the second solution were mixed (the molar ratio of the thiol groups to the maleimide groups was 0.36) under a pH value of 7.8 (an alkaline condition) to prepare a hydrogel (GAEG02). Also, the gelation time was less than 2 minutes.

In this comparative example, the mixture of the first solution and the second solution was inhomogeneous during the preparation due to rapid gelation (less than 2 minutes). In tests of drug-release behavior, release of Herceptin from the hydrogels was incomplete. The hydrogels (GAEG01 and GAEG02) merely released Herceptin for about 28 days. In addition, in tests of drug structure and activity, the integrity of the molecular structure of Herceptin was seriously damaged (formation of numerous Herceptin fragments) and the activity thereof was lost (the binding ability to antigen was significantly reduced).

### Example 35

### Effect of acidic pH on encapsulation and dissolution of the drug-loaded hydrogels

The hydrogel GAEG13 was prepared by Example 25. The hydrogel GAEG14 was prepared by Example 26. The hydrogel GAEG15 was prepared by Example 27. Effect of acidic pH on encapsulation and dissolution of the drug-loaded hydrogels (GAEG13, GAEG14 and GAEG15) was tested.

In this example, the maximum drug loading concentration of the hydrogels (GAEG13, GAEG14 and GAEG15) achieved 205mg/mL. The mixture (hydrogel/drug) of the first solution and the second solution was homogeneous during the preparation due to sufficient gelation time (2-17 minutes). In tests of drug release behavior, release of Herceptin from the hydrogels was complete. The hydrogels (GAEG13, GAEG14 and GAEG15) released Herceptin for up to about 50 days (i.e. the hydrogels with sustained-release capacity). In addition, in tests of drug structure and activity, the integrity of the molecular structure of Herceptin was maintained, for example, to Day 35, there was still no Herceptin fragment formation. The biological activity thereof was thus maintained (the binding ability to antigen was still high).

### Example 36

### The dissolution effect of the drug complex-loaded hydrogel (1)

0.01848g of Herceptin powder in 50uL MOPS buffer and 0.001g of polyglutamic acid (PGA) containing maleimide groups (Mw: 200-400kDa, DS (grafting ratio): 11.3%) in 50uL MOPS buffer were blended to form a complex solution and centrifuged to remove the 30uL supernatant. Next, 0.001g of polyethylene glycol (PEG) containing thiol groups (Mw: 5kDa, 4-arm type) was dissolved in 30µL of 0.9% NaCl solution and blended with the complex solution to prepare a hydrogel (PGA01) (final drug concentration: 184.8mg/mL). In this preparation, the molar ratio of the thiol groups to the maleimide groups was 1.0, and the pH of the blending solution was 6.0. By visual confirmation, the gelation was successful and took 5 minutes.

In this example, in tests of drug release behavior, release of Herceptin from the hydrogel was complete. The hydrogel (PGA01) released Herceptin for up to about 42 days (i.e. the hydrogel with sustained-release capacity). In addition, in tests of drug structure and activity, the integrity of the molecular structure of Herceptin was maintained, for example, to Day 28, there was still no Herceptin fragment/aggregation formation. The biological activity thereof was thus maintained (the binding ability to antigen was still high).

### Example 37

### The dissolution effect of the drug complex-loaded hydrogels (2)

A Herceptin/Zn complex solution having concentration of 128.0mg/mL was prepared using 0.9% NaCl solution as a buffer solution. A proper amount of polyglutamic acid (PGA) containing maleimide groups (Mw: 1,000kDa, DS (grafting ratio): 12.5%) and polyethylene glycol (PEG) containing thiol groups (Mw: 5kDa, 4-arm type) were dissolved in the Herceptin/Zn complex solution to prepare Herceptin hydrogels with PGA concentration of 1.0wt% and PEG concentration of 0.75wt% (the molar ratio of the thiol groups to the maleimide groups was 0.7) under a pH value of 5.0 (an acidic condition) to prepare a hydrogel (GAEGZ001). The gelation time was more than 30 minutes.

A Herceptin/Zn complex solution having concentration of 208.0mg/mL was prepared using 0.5M histidine solution as a buffer solution. A proper amount of polyglutamic acid (PGA) containing maleimide groups (Mw: 1,000kDa, DS (grafting ratio): 12.5%) and polyethylene glycol (PEG) containing thiol groups (Mw: 5kDa, 4-arm type) were dissolved in the Herceptin/Zn complex solution to prepare Herceptin hydrogels with PGA concentration of 1.3wt% and PEG concentration of 0.8wt% (the molar ratio of the thiol groups to the maleimide groups was 0.6) under a pH value of 4.3 (an acidic condition) to prepare a hydrogel (GAEGZ002). The gelation time was 5-10 minutes.

In this example, the hydrogel (GAEGZ002) had a drug loading concentration of up to 208mg/mL. The hydrogel (GAEGZ001) released Herceptin for up to about 42 days, and the hydrogel (GAEGZ002) released Herceptin for even up to about 70 days (i.e. the hydrogels with strong sustained-release capacity). It is because Herceptin and Zn interact to form a chelate, resulting in enhancement of the sustained-release effect. In addition, in tests of drug structure and activity, the integrity and stability of the molecular structure of Herceptin was maintained, for example, the monomer of released Herceptin was over 90%. The biological activity thereof was thus maintained (the binding ability to antigen was still high).

### Example 38

### The release behavior of the drug-loaded hydrogels

A Herceptin solution having a concentration of 10.6mg/mL was prepared using 0.9% NaCl solution as a buffer solution. A proper amount of polyglutamic acid (PGA) containing maleimide groups (Mw: 200-400kDa, DS (grafting ratio): 11.3%) was then dissolved in the Herceptin solution to prepare a first solution having a concentration of 3.0wt%. A proper amount of polyethylene glycol (PEG) containing thiol groups (Mw: 5kDa, 4-arm type) was then dissolved in the Herceptin solution to prepare a second solution having a concentration of 3.0wt%. The first solution and the second solution were then mixed (the molar ratio of the thiol groups to the maleimide groups was 1.0) under a pH value of 6.0 (an acidic condition) to prepare a hydrogel. The gelation time was 10-15 minutes.

A Herceptin solution having a concentration of 128.6mg/mL was prepared using 0.9% NaCl solution as a buffer solution. A proper amount of polyglutamic acid (PGA) containing maleimide groups (Mw: 200-400kDa, DS (grafting ratio): 11.3%) was then dissolved in the Herceptin solution to prepare a first solution having a concentration of 3.0wt%. A proper amount of polyethylene glycol (PEG) containing thiol groups (Mw: 5kDa, 4-arm type) was then dissolved in the Herceptin solution to prepare a second solution having a concentration of 3.0wt%. The first solution and the second solution were then mixed (the molar ratio of the thiol groups to the maleimide groups was 1.0) under a pH value of 6.0 (an acidic condition) to prepare a hydrogel (GAEG11). The gelation time was 4 minutes.

A Herceptin solution having a concentration of 205.0mg/mL was prepared using 0.9% NaCl solution as a buffer solution. A proper amount of polyglutamic acid (PGA) containing maleimide groups (Mw: 200-400kDa, DS (grafting ratio): 11.3%) was then dissolved in the Herceptin solution to prepare a first solution having a concentration of 3.0wt%. A proper amount of polyethylene glycol (PEG) containing thiol groups (Mw: 5kDa, 4-arm type) was then dissolved in the Herceptin solution to prepare a second solution having a concentration of 3.0wt%. The first solution and the second solution were then mixed (the molar ratio of the thiol groups to the maleimide groups was 1.0) under a pH value of 6.0 (an acidic condition) to prepare a hydrogel (GAEG15). The gelation time was 4 minutes.

In this example, in tests of drug release behavior, the results indicate that the higher the drug loading concentration, the longer the drug release period. When the drug loading concentration was 128.6mg/mL or 205.0mg/mL, the drug release period was up to about 42 days.

### Example 39

### The release behavior of the drug complex-loaded hydrogels

A Herceptin/Zn complex solution having concentration of 9.3mg/mL was prepared using 0.5M histidine solution as a buffer solution. A proper amount of polyglutamic acid (PGA) containing maleimide groups (Mw: 200-400kDa, DS (grafting ratio: 9.0%) and polyethylene glycol (PEG) containing thiol groups (Mw: 5kDa, 4-arm type) were dissolved in the Herceptin/Zn complex solution to prepare Herceptin hydrogels with PGA concentration of 1.5wt% and PEG concentration of 1.5wt% (the molar ratio of the thiol groups to the maleimide groups was 1.0) under a pH value of 4.1 (an acidic condition) to prepare a hydrogel (GAEGZ007). The gelation time was 50-60 minutes.

A Herceptin/Zn complex solution having concentration of 93.0mg/mL was prepared using 0.5M histidine solution as a buffer solution. A proper amount of polyglutamic acid (PGA) containing maleimide groups (Mw: 200-400kDa, DS (grafting ratio: 9.0%) and polyethylene glycol (PEG) containing thiol groups (Mw: 5kDa, 4-arm type) were dissolved in the Herceptin/Zn complex solution to prepare Herceptin hydrogels with a PGA concentration of 1.5wt% and a PEG concentration of 1.5wt% (the molar ratio of the thiol groups to the maleimide groups was 1.0) under a pH value of 4.2 (an acidic condition) to prepare a hydrogel (GAEGZ008). The gelation time was 35-40 minutes.

A Herceptin/Zn complex solution having concentration of 171.7mg/mL was prepared using 0.5M histidine solution as a buffer solution. A proper amount of polyglutamic acid (PGA) containing maleimide groups (Mw: 200-400kDa, DS (grafting ratio: 9.0%) and polyethylene glycol (PEG) containing thiol groups (Mw: 5kDa, 4-arm type) were dissolved in the Herceptin/Zn complex solution to prepare Herceptin hydrogels with PGA concentration of 1.5wt% and PEG concentration of 1.5wt% (the molar ratio of the thiol groups to the maleimide groups was 1.0) under a pH value of 4.3 (an acidic condition) to prepare a hydrogel (GAEGZ009). The gelation time was 25-30 minutes. In this example, in tests of drug release behavior, the results indicate that the higher the drug loading concentration, the longer the drug release period. When the drug loading concentration was 93.0mg/mL or 171.7mg/mL, the drug release period was up to about 35 days.

### Example 40

### Effect of acidic pH on encapsulation and dissolution of the drug-loaded hydrogels

An Etanercept solution having a concentration of 141.3mg/mL was prepared. A proper amount of polyglutamic acid (PGA) containing maleimide groups (Mw: 200-400kDa, DS (grafting ratio): 5.0%) was dissolved in the Etanercept solution to prepare a first solution having a concentration of 4.1 wt%. A proper amount of polyethylene glycol (PEG) containing thiol groups (Mw: 5kDa, 4-arm type) was dissolved in the Etanercept solution to prepare a second solution having a concentration of 1.5 wt%. The first solution and the second solution were mixed (the molar ratio of the thiol groups to the maleimide groups was 0.8) under a pH value of 6.3 (an acidic condition) to prepare a hydrogel. The gelation time was 90-120 minutes.

Next, a proper amount of polyglutamic acid (PGA) containing maleimide groups (Mw: 200-400kDa, DS (grafting ratio): 5.0%) was dissolved in the Etanercept solution to prepare a first solution having a concentration of 4.9 wt%. A proper amount of polyethylene glycol (PEG) containing thiol groups (Mw: 5kDa, 4-arm type) was dissolved in the Etanercept solution to prepare a second solution having a concentration of 1.5 wt%. The first solution and the second solution were mixed (the molar ratio of the thiol groups to the maleimide groups was 0.67) under a pH value of 6.3 (an acidic condition) to prepare a hydrogel. The gelation time was 45-90 minutes.

Next, a proper amount of polyglutamic acid (PGA) containing maleimide groups (Mw: 200-400kDa, DS (grafting ratio): 5.0%) was dissolved in the Etanercept solution to prepare a first solution having a concentration of 5.7wt%. A proper amount of polyethylene glycol (PEG) containing thiol groups (Mw: 5kDa, 4-arm type) was dissolved in the Etanercept solution to prepare a second solution having a concentration of 1.5wt%. The first solution and the second solution were mixed (the molar ratio of the thiol groups to the maleimide groups was 0.57) under a pH value of 6.3 (an acidic condition) to prepare a hydrogel. The gelation time was 30-45 minutes.

Next, a proper amount of polyglutamic acid (PGA) containing maleimide groups (Mw: 200-400kDa, DS (grafting ratio): 5.0%) was dissolved in the Etanercept solution to prepare a first solution having a concentration of 6.5wt%. A proper amount of polyethylene glycol (PEG) containing thiol groups (Mw: 5kDa, 4-arm type) was dissolved in the Etanercept solution to prepare a second solution having a concentration of 1.5wt%. The first solution and the second solution were mixed (the molar ratio of the thiol groups to the maleimide groups was 0.5) under a pH value of 6.3 (an acidic condition) to prepare a hydrogel. The gelation time was 30-45 minutes.

In this example, the maximum drug loading concentration of the hydrogels achieved 141.3mg/mL. The mixture (hydrogel/drug) of the first solution and the second solution was homogeneous during the preparation due to sufficient gelation time (30-120 minutes). In tests of drug release behavior, release of Etanercept from the hydrogels was complete. The hydrogels released Etanercept for up to about 30 days (i.e. the hydrogels with sustained-release capacity). In addition, in tests of drug structure and activity, the integrity of the molecular structure of Etanercept was maintained. The biological activity thereof was thus maintained (the binding ability to antigen was still high).

### Example 41

### Effect of acidic pH on encapsulation and dissolution of the drug-loaded hydrogels

A HSA (human serum albumin) solution having a concentration of 100 mg/mL was prepared. A proper amount of polyglutamic acid (PGA) containing maleimide groups (Mw: 200-400kDa, DS (grafting ratio): 12.1%) was dissolved in the HSA solution to prepare a first solution having a concentration of 2.0wt%. A proper amount of polyethylene glycol (PEG) containing thiol groups (Mw: 5kDa, 4-arm type) was dissolved in the HSA solution to prepare a second solution having a concentration of 2.0wt%. The first solution and the second solution were mixed (the molar ratio of the thiol groups to the maleimide groups was 1.0) under a pH value of 5.0-6.15 (an acidic condition) to prepare a hydrogel (A). The gelation time was 8-12 minutes.

A HSA (human serum albumin) solution having a concentration of 200mg/mL was prepared. Next, a proper amount of polyglutamic acid (PGA) containing maleimide groups (Mw: 200-400kDa, DS (grafting ratio): 12.1%) was dissolved in the HSA solution to prepare a first solution having a concentration of 2.0wt%. A proper amount of polyethylene glycol (PEG) containing thiol groups (Mw: 5kDa, 4-arm type) was dissolved in the HSA solution to prepare a second solution having a concentration of 2.0wt%. The first solution and the second solution were mixed (the molar ratio of the thiol groups to the maleimide groups was 1.0) under a pH value of 5.0-6.15 (an acidic condition) to prepare a hydrogel (B). The gelation time was 2.5-4.2 minutes.

A HSA (human serum albumin) solution having a concentration of 300mg/mL was prepared. Next, a proper amount of polyglutamic acid (PGA) containing maleimide groups (Mw: 200-400kDa, DS (grafting ratio): 12.1%) was dissolved in the HSA solution to prepare a first solution having a concentration of 2.0wt%. A proper amount of polyethylene glycol (PEG) containing thiol groups (Mw: 5kDa, 4-arm type) was dissolved in the HSA solution to prepare a second solution having a concentration of 2.0wt%. The first solution and the second solution were mixed (the molar ratio of the thiol groups to the maleimide groups was 1.0) under a pH value of 6.15 (an acidic condition) to prepare a hydrogel (C). The gelation time was less than 1 minute.

In this example, the maximum drug loading concentration of the hydrogels (A, B and C) achieved 100-300mg/mL. The mixture (hydrogel/drug) of the first solution and the second solution was homogeneous during the preparation due to sufficient gelation time. In addition, in tests of drug structure, the integrity and stability of the molecular structure of HSA was maintained, for example, the monomer of released HSA from the hydrogels (A, B and C) was respectively 94.1%, 92.8% and 92.6%.

### Comparative Example 3

### Effect of alkaline pH on encapsulation and dissolution of the drug-loaded hydrogels

A HSA (human serum albumin) solution having a concentration of 50mg/mL was prepared. A proper amount of polyglutamic acid (PGA) containing maleimide groups (Mw: 200-400kDa, DS (grafting ratio): 12.1%) was dissolved in the HSA solution to prepare a first solution having a concentration of 2.0wt%. A proper amount of polyethylene glycol (PEG) containing thiol groups (Mw: 5kDa, 4-arm type) was dissolved in the HSA solution to prepare a second solution having a concentration of 2.0wt%. The first solution and the second solution were mixed (the molar ratio of the thiol groups to the maleimide groups was 1.0) under a pH value of 8.18 (an alkaline condition) to prepare a hydrogel (D). However, the hydrogel was gelatinized immediately.

In this comparative example, the mixture of the first solution and the second solution was inhomogeneous during the preparation due to rapid gelation (i.e. the hydrogel was gelatinized immediately). In addition, in tests of drug structure, the integrity of the molecular structure of HSA was seriously damaged (formation of numerous HSA fragments). The monomer of released HSA from the hydrogel was merely 75.8%.

### Example 42

### Effect of acidic pH on encapsulation and dissolution of the drug-loaded hydrogels

A Liraglutide solution having a concentration of 20mg/mL was prepared. A proper amount of polyglutamic acid (PGA) containing maleimide groups (Mw: 200-400kDa, DS (grafting ratio): 12.1%) was dissolved in the Liraglutide solution to prepare a first solution having a concentration of 2.0wt%. A proper amount of polyethylene glycol (PEG) containing thiol groups (Mw: 5kDa, 4-arm type) was dissolved in the Liraglutide solution to prepare a second solution having a concentration of 2.0wt%. The first solution and the second solution were mixed (the molar ratio of the thiol groups to the maleimide groups was 1.0) under a pH value of 5.0 (an acidic condition) to prepare a hydrogel (A). The gelation time was 8-12 minutes.

A Liraglutide solution having a concentration of 20mg/mL was prepared. Next, a proper amount of polyglutamic acid (PGA) containing maleimide groups (Mw: 300kDa, DS (grafting ratio): 12.1%) was dissolved in the Liraglutide solution to prepare a first solution having a concentration of 3.0wt%. A proper amount of polyethylene glycol (PEG) containing thiol groups (Mw: 5kDa, 4-arm type) was dissolved in the Liraglutide solution to prepare a second solution having a concentration of 3.0wt%. The first solution and the second solution were mixed (the molar ratio of the thiol groups to the maleimide groups was 1.0) under a pH value of 5.0 (an acidic condition) to prepare a hydrogel (B). The gelation time was 2.5-4.2 minutes.

In this example, the mixture (hydrogel/drug) of the first solution and the second solution was homogeneous during the preparation due to sufficient gelation time (2-12 minutes). In addition, in tests of drug structure, the integrity and stability of the molecular structure of Liraglutide was maintained, for example, the monomer of released Liraglutide from the hydrogels (A and B) was 100%.

### Example 43

### The in-vivo pharmacokinetics (PK) study of the drug-loaded hydrogel

A "Herceptin" solution having a concentration of 20.0mg/mL was first prepared as a control group.

Another Herceptin solution having concentration of 20.0mg/mL was prepared. Next, a proper amount of polyglutamic acid (PGA) containing maleimide groups (Mw: 200-400kDa, DS (grafting ratio): 11.4%) was dissolved in the Herceptin solution to prepare a first solution having a concentration of 3.0wt%. A proper amount of polyethylene glycol (PEG) containing thiol groups (Mw: 5kDa, 4-arm type) was then dissolved in the Herceptin solution to prepare a second solution having a concentration of 3.0wt%. The first solution and the second solution were then mixed (the molar ratio of the thiol groups to the maleimide groups was 1.0) under a pH value of 6.0 (an acidic condition) to prepare a "Herceptin-loaded hydrogel" as an experimental group.

Pharmacokinetics (PK) experiments were carried out with "Herceptin" (control group) and "Herceptin-loaded hydrogel" (experimental group) in rats. The dose thereof was 50mg/kg. The drug concentration in plasma was respectively measured on day 7, day 14, day 21, day 28, and day 35 after dosing to build serum concentration-time profiles. In accordance with the data of the serum concentration-time profiles, pharmacokinetics parameters, such as Tₘₐₓ, Cₘₐₓ, T_{1/2}, AUC_{D35} and BA (bioavailability), were obtained and are shown in Table 2.

**Table 2**

| Pharmacokinetics (PK) parameters | | Herceptin | Herceptin-loaded hydrogel |
|---|---|---|---|
| Tₘₐₓ | day | 3.0 ± 0 | 6.7 ± 0.6 |
| Cₘₐₓ | µg/mL | 272 ± 25 | 154 ± 18 |
| T_{1/2} | day | 2.6 ± 0.4 | 7 ± 3.7 |
| AUC_{D35} | day^{∗}µg/mL | 3,079 ± 100 | 2,522 ± 215 |
| BA | % | 100% | 82% |

The results indicate that, for the Herceptin-loaded hydrogel, Cₘₐₓ thereof is about 60% of the original Herceptin, Tₘₐₓ thereof delayed 2 times, and T_{1/2} thereof extended 2.7 times. Therefore, the Herceptin-loaded hydrogel possesses sustained-release capacity.

### Example 44

### The in-vivo pharmacokinetics (PK) study of the drug complex-loaded hydrogel

A "Herceptin" solution having a concentration of 20.0mg/mL was first prepared as a control group.

A Herceptin/Zn complex solution having concentration of 20.0mg/mL was prepared using 0.9% NaCl solution as a solution. A proper amount of polyglutamic acid (PGA) containing maleimide groups (Mw: 200-400kDa, DS (grafting ratio: 11.4%) and polyethylene glycol (PEG) containing thiol groups (Mw: 5kDa, 4-arm type) were dissolved in the Herceptin/Zn complex solution with PGA concentration of 1.5wt% and PEG concentration of 1.5wt% (the molar ratio of the thiol groups to the maleimide groups was 1.0) under a pH value of 5.8 (an acidic condition) to prepare a "Herceptin/Zn complex-loaded hydrogel (Her/Zn-hydrogel)" as an experimental group. The gelation time was 40-50 minutes.

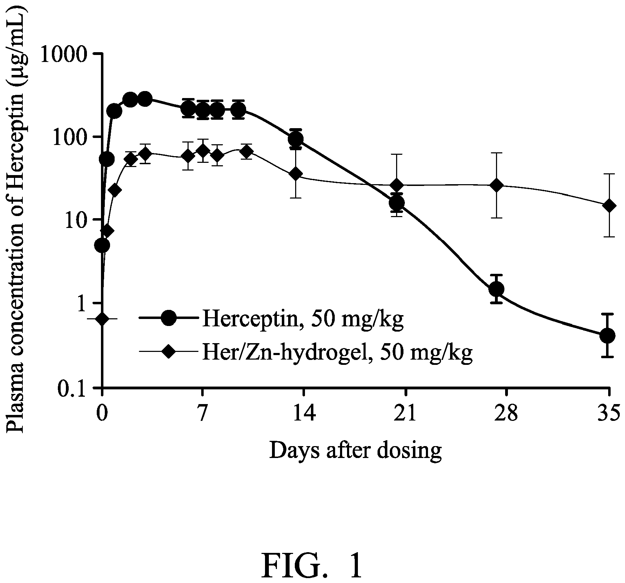

Pharmacokinetics (PK) experiments were carried out with "Herceptin" (control group) and "Herceptin/Zn complex-loaded hydrogel (Her/Zn-hydrogel)" (experimental group) in rats. The dose thereof was 50mg/kg. The drug concentration in plasma was respectively measured on day 7, day 14, day 21, day 28, and day 35 after dosing to build serum concentration-time profiles. In accordance with the data of the serum concentration-time profiles, pharmacokinetics parameters, such as Tₘₐₓ, Cₘₐₓ, T_{1/2}, AUC_{D35} and BA (bioavailability), were obtained and are shown in Table 3 and FIG. 1.

**Table 3**

| Pharmacokinetics (PK) parameters | | Herceptin | Herceptin/Zn complex-loaded hydrogel |
|---|---|---|---|
| Tₘₐₓ | day | 3.0 ± 0 | 10 ± 3.5 |
| Cₘₐₓ | µg/mL | 272 ± 25 | 76 ± 17 |
| T_{1/2} | day | 2.6 ± 0.4 | 11 ± 12 |
| AUC_{D35} | day^{∗}µg/mL | 3,079 ± 100 | 1,128 ± 448 |
| BA | % | 100% | 37% |

The results indicate that, for the Herceptin/Zn complex-loaded hydrogel, Cₘₐₓ thereof is about 30% of the original Herceptin, Tₘₐₓ thereof delayed 3 times, and T_{1/2} thereof extended 4.2 times. Therefore, the Herceptin/Zn complex-loaded hydrogel possesses sustained-release capacity.

### Example 45

### The in-vivo pharmacodynamics (PD) study of the drug-loaded hydrogel

A "Dulbecco's phosphate-buffered saline (DPBS)" solution was provided as a control group.

A "Herceptin" solution having a concentration of 10mg/mL was prepared as a first experimental group.

Another Herceptin solution having a concentration of 10mg/mL was prepared. Next, a proper amount of polyglutamic acid (PGA) containing maleimide groups (Mw: 200-400kDa, DS (grafting ratio): 11.5%) was dissolved in the Herceptin solution to prepare a first solution having a concentration of 3.0wt%. A proper amount of polyethylene glycol (PEG) containing thiol groups (Mw: 5kDa, 4-arm type) was then dissolved in the Herceptin solution to prepare a second solution having a concentration of 3.0wt%. The first solution and the second solution were then mixed (the molar ratio of the thiol groups to the maleimide groups was 1.0) under a pH value of 6.0 (an acidic condition) to prepare a "Herceptin-loaded hydrogel" as a second experimental group.

Pharmacodynamics (PD) experiments were carried out with "DPBS" (control group), "Herceptin" (first experimental group), and "Herceptin-loaded hydrogel" (second experimental group) in BT474 breast cancer mouse. The dose of "Herceptin" and "Herceptin-loaded hydrogel" was 50mg/kg. The tumor volume (mm³) was respectively measured on specific days after dosing to build tumor-volume variation curves. In accordance with the data of the tumor-volume variation curves, the tumor growth inhibition (TGI) was calculated and is shown in Table 4.

**Table 4**

| Day 28 after dosing | TGI (%± SEM) | BW (%±SEM) | N |
|---|---|---|---|
| DPBS | 0.0 | 107.1±1.6 | 8 |
| Herceptin | 52.7±22.2 | 100.6±4.1 | 7 |
| Herceptin-loaded hydrogel | 44.2±32.0 | 102.5±3.1 | 8 |

The results of tumor volume variation on Day 28 after dosing indicate that, compared to DPBS, Herceptin (dose: 50mg/kg) applied by subcutaneous injection is able to inhibit tumor growth, and the TGI thereof was calculated as 52.7%.

The TGI of the Herceptin-loaded hydrogel is similar to that of Herceptin on Day 28 after dosing.

There was no significant change in body weight (BW) of the mouse during the experiments.

### Example 46

### The in-vivo pharmacodynamics (PD) study of the drug complex-loaded hydrogel

A "Dulbecco's phosphate-buffered saline (DPBS)" solution was provided as a control group.

A Herceptin/Zn complex solution having concentration of 10.0 mg/mL was prepared using 0.9% NaCl solution as a solution. A proper amount of polyglutamic acid (PGA) containing maleimide groups (Mw: 200-400kDa, DS (grafting ratio: 11.5%) and polyethylene glycol (PEG) containing thiol groups (Mw: 5kDa, 4-arm type) were dissolved in the Herceptin/Zn complex solution with PGA concentration of 1.5wt% and PEG concentration of 1.5wt% (the molar ratio of the thiol groups to the maleimide groups was 1.0) under a pH value of 5.9 (an acidic condition) to prepare a "Herceptin/Zn complex-loaded hydrogel (Her/Zn-hydrogel)" as an experimental group. The gelation time was around 40 minutes.

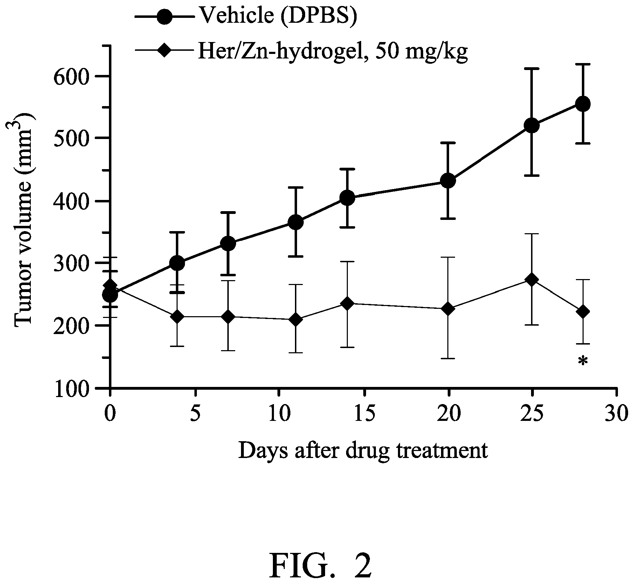

Pharmacodynamics (PD) experiments were carried out with "DPBS" (control group) and "Herceptin/Zn complex-loaded hydrogel (Her/Zn-hydrogel)" (experimental group) in BT474 breast cancer mouse. The dose of "Herceptin/Zn-hydrogel" was 50mg/kg. The tumor volume (mm³) was respectively measured on specific days after dosing to build tumor-volume variation curves. In accordance with the data of the tumor-volume variation curves, the tumor growth inhibition (TGI) was calculated and is shown in Table 5 and FIG. 2.

**Table 5**

| Day 28 after dosing | TGI (%± SEM) | BW (%±SEM) | N |
|---|---|---|---|
| DPBS | 0.0 | 107.1±1.6 | 8 |
| Her/Zn-hydrogel | 121.9±35.0 | 98.8±2.9 | 7 |

The results of tumor volume variation on Day 28 after dosing indicate that the Herceptin/Zn complex-loaded hydrogel is able to significantly inhibit tumor growth, and the TGI thereof was calculated as 121.9%.

There was no significant change in body weight (BW) of the mouse during the experiments.

The present disclosure provides a novel hydrogel composition which comprises polyglutamic acid (PGA) containing maleimide (MA) groups, and polyethylene glycol (PEG) containing thiol (SH) groups. Specifically, the hydrogel composition has an acidic pH value ranging from about 4.0 to about 6.5. The hydrogel can be applied in drug delivery systems. The hydrogel has the ability to carry high-dose drugs, with a maximum drug loading concentration up to about 300mg/mL. The hydrogel is able to regulate the release behavior of drugs, for example, the sustained-release period is up to at least 35 days *in vitro.* Specifically, for drug complex-loaded hydrogels, Cₘₐₓ thereof is about 30% of the drug uncovered by the hydrogel, Tₘₐₓ thereof delayed 3 times, and T_{1/2} thereof extended 4.2 times *in vivo.* The integrity and stability of the molecular structure and biological activity of drugs are maintained by the hydrogel. In addition, the hydrogel is able to encapsulate multivariate drugs, for example, macro-molecular drugs such as antibodies or proteins or peptides, or hydrophilic or hydrophobic small molecules.

## Claims

1. A hydrogel composition, comprising:
polyglutamic acid (PGA) containing maleimide groups; and
polyethylene glycol (PEG) containing terminal thiol groups, wherein the hydrogel composition has a pH value ranging from 4.0 to 6.5.

2. The hydrogel composition as claimed in claim 1, wherein the polyglutamic acid (PGA) containing maleimide groups has a molecular weight ranging from 10kDa to 1,000kDa.

3. The hydrogel composition as claimed in one of claims 1 and 2, wherein the polyglutamic acid (PGA) containing maleimide groups has a grafting ratio ranging from 5% to 40%.

4. The hydrogel composition as claimed in one of claims 1, 2 and 3, wherein the polyglutamic acid (PGA) containing maleimide groups has a concentration ranging from 0.75wt% to 10wt% in the hydrogel composition.

5. The hydrogel composition as claimed in one of claims 1, 2, 3 and 4, wherein the polyglutamic acid (PGA) containing maleimide groups is free of thiol groups.

6. The hydrogel composition as claimed in one of claims 1, 2, 3, 4 and 5, wherein the polyethylene glycol (PEG) containing terminal thiol groups has a molecular weight ranging from 2kDa to 20kDa.

7. The hydrogel composition as claimed in one of claims 1, 2, 3, 4, 5 and 6, wherein the polyethylene glycol (PEG) containing terminal thiol groups has a concentration ranging from 0.75wt% to 10wt% in the hydrogel composition.

8. The hydrogel composition as claimed in one of claims 1, 2, 3, 4, 5, 6 and 7, wherein the polyethylene glycol (PEG) containing terminal thiol groups is 4-arm type, 8-arm type or Y-shape.

9. The hydrogel composition as claimed in one of claims 1, 2, 3, 4, 5, 6, 7 and 8, wherein the polyethylene glycol (PEG) containing terminal thiol groups is free of maleimide groups.

10. The hydrogel composition as claimed in one of claims 1, 2, 3, 4, 5, 6, 7, 8 and 9, wherein the polyethylene glycol (PEG) containing terminal thiol groups and the polyglutamic acid (PGA) containing maleimide groups have a molar ratio of the thiol group to the maleimide group ranging from 0.2 to 5.0.

11. The hydrogel composition as claimed in one of claims 1, 2, 3, 4, 5, 6, 7, 8, 9 and 10, wherein the polyethylene glycol (PEG) containing terminal thiol groups and the polyglutamic acid (PGA) containing maleimide groups have a molar ratio of the thiol group to the maleimide group ranging from 1.0 to 1.5.

12. A drug delivery system, comprising:
a hydrogel composition as claimed in one of claims 1, 2, 3, 4, 5, 6, 7, 8, 9, 10 and 11; and
a pharmaceutically active ingredient encapsulated in the hydrogel composition.

13. The drug delivery system as claimed in claim 12, wherein the pharmaceutically active ingredient is selected from the group consisting of growth factors, hormones, peptides, proteins, antibodies, hydrophilic small molecules and hydrophobic small molecules.

14. The drug delivery system as claimed in one of claims 12 and 13, wherein the pharmaceutically active ingredient is selected from the group consisting of intact antibodies and antibody fragments.

15. The drug delivery system as claimed in one of claims 12, 13 and 14, wherein the pharmaceutically active ingredient is selected from the group consisting of murine antibodies, chimeric antibodies, humanized antibodies, and human antibodies.

16. The drug delivery system as claimed in one of claims 12 and 13, wherein the pharmaceutically active ingredient is selected from the group consisting of antineoplastic agents, antipsychotics, analgesics and antibiotics.

17. The drug delivery system as claimed in one of claims 12, 13, 14, 15 and 16, wherein the pharmaceutically active ingredient is further associated with a polymer, metal, charged compounds or charged particles to form a complex thereof.

18. The drug delivery system as claimed in claim 17, wherein the polymer comprises polyglutamic acid (PGA), hyaluronic acid, chitosan or dextran.

19. The drug delivery system as claimed in claim 17, wherein the metal comprises zinc, calcium, magnesium or iron.

20. The drug delivery system as claimed in claim 17, wherein the complex has a size ranging from 10nm to 100µm.

21. The drug delivery system as claimed in one of claims 12, 17 and 20, wherein the pharmaceutically active ingredient or the complex has a concentration ranging from 1mg/mL to 300mg/mL.

## Patentansprüche

1. Hydrogelzusammensetzung, mit:
Polyglutaminsäure (PGA), die Maleimidgruppen enthält; und
Polyethylenglykol (PEG), das endständige Thiolgruppen enthält, wobei die Hydrogelzusammensetzung einen pH-Wert im Bereich von 4,0 bis 6,5 aufweist.

2. Hydrogelzusammensetzung nach Anspruch 1, wobei die Maleimidgruppen enthaltende Polyglutaminsäure (PGA) ein Molekulargewicht im Bereich von 10 kDa bis 1000 kDa aufweist.

3. Hydrogelzusammensetzung nach Anspruch 1 oder 2, wobei die Maleimidgruppen enthaltende Polyglutaminsäure (PGA) ein Pfropfverhältnis im Bereich von 5% bis 40% aufweist.

4. Hydrogelzusammensetzung nach einem der Ansprüche 1, 2 und 3, wobei die Maleinimidgruppen enthaltende Polyglutaminsäure (PGA) eine Konzentration im Bereich von 0,75 Gew.-% bis 1 Gew.-% in der Hydrogelzusammensetzung aufweist.

5. Hydrogelzusammensetzung nach einem der Ansprüche 1, 2, 3 und 4, wobei die Maleimidgruppen enthaltende Polyglutaminsäure (PGA) frei von Thiolgruppen ist.

6. Hydrogelzusammensetzung nach einem der Ansprüche 1, 2, 3, 4 und 5, wobei das endständige Thiolgruppen enthaltende Polyethylenglykol (PEG) ein Molekulargewicht im Bereich von 2 kDa bis 20 kDa aufweist.

7. Hydrogelzusammensetzung nach einem der Ansprüche 1, 2, 3, 4, 5 und 6, wobei das endständige Thiolgruppen enthaltende Polyethylenglykol (PEG) eine Konzentration im Bereich von 0,75 Gew.-% bis 10 Gew.-% in der Hydrogelzusammensetzung aufweist.

8. Hydrogelzusammensetzung nach einem der Ansprüche 1, 2, 3, 4, 5, 6 und 7, wobei das endständige Thiolgruppen enthaltende Polyethylenglykol (PEG) vom 4-armigen Typ, 8-armigen Typ oder Y-förmig ist.

9. Hydrogelzusammensetzung nach einem der Ansprüche 1, 2, 3, 4, 5, 6, 7 und 8, wobei das endständige Thiolgruppen enthaltende Polyethylenglykol (PEG) frei von Maleinimidgruppen ist.

10. Hydrogelzusammensetzung nach einem der Ansprüche 1, 2, 3, 4, 5, 6, 7, 8 und 9, wobei das endständige Thiolgruppen enthaltende Polyethylenglykol (PEG) und die Maleinimidgruppen enthaltende Polyglutaminsäure (PGA) ein Molverhältnis der Thiolgruppe zur Maleinimidgruppe im Bereich von 0,2 bis 5,0 aufweisen.

11. Hydrogelzusammensetzung nach einem der Ansprüche 1, 2, 3, 4, 5, 6, 7, 8, 9 und 10, wobei das endständigen Thiolgruppen enthaltenden Polyethylenglykol (PEG) und die Maleinimidgruppen enthaltenden Polyglutaminsäure (PGA) ein Molverhältnis der Thiolgruppe zur Maleinimidgruppe im Bereich von 1,0 bis 1,5 aufweisen.

12. Arzneimittelabgabesystem, mit:
einer Hydrogelzusammensetzung nach einem der Ansprüche 1, 2, 3, 4, 5, 6, 7, 8, 9, 10 und 11; und
einem in der Hydrogelzusammensetzung eingekapselten pharmazeutischen Wirkstoff.

13. Arzneimittelabgabesystem nach Anspruch 12, wobei der pharmazeutische Wirkstoff ausgewählt ist aus der Gruppe bestehend aus Wachstumsfaktoren, Hormonen, Peptiden, Proteinen, Antikörpern, hydrophilen kleinen Molekülen und hydrophoben kleinen Molekülen.

14. Arzneimittelabgabesystem nach Anspruch 12 oder 13, wobei der pharmazeutische Wirkstoff ausgewählt ist aus der Gruppe bestehend aus intakten Antikörpern und Antikörperfragmenten.

15. Arzneimittelabgabesystem nach einem der Ansprüche 12, 13 und 14, wobei der pharmazeutische Wirkstoff ausgewählt ist aus der Gruppe bestehend aus murinen Antikörpern, chimären Antikörpern, humanisierten Antikörpern und humanen Antikörpern.

16. Arzneimittelabgabesystem nach Anspruch 12 oder 13,
wobei der pharmazeutische Wirkstoff ausgewählt ist aus der Gruppe bestehend aus antineoplastischen Mitteln, Antipsychotika, Analgetika und Antibiotika.

17. Arzneimittelabgabesystem nach einem der Ansprüche 12, 13, 14, 15 und 16, wobei der pharmazeutische Wirkstoff ferner mit einem Polymer, einem Metall, geladenen Verbindungen oder geladenen Teilchen assoziiert ist, um einen Komplex davon zu bilden.

18. Arzneimittelabgabesystem nach Anspruch 17, wobei das Polymer Polyglutaminsäure (PGA), Hyaluronsäure, Chitosan oder Dextran aufweist.

19. Arzneimittelabgabesystem nach Anspruch 17, wobei das Metall Zink, Calcium, Magnesium oder Eisen aufweist.

20. Arzneimittelabgabesystem nach Anspruch 17, wobei der Komplex eine Größe im Bereich von 10 nm bis 100 µm aufweist.

21. Arzneimittelabgabesystem nach einem der Ansprüche 12, 17 und 20, wobei der pharmazeutische Wirkstoff oder der Komplex eine Konzentration im Bereich von 1 mg/ml bis 300 mg/ml aufweist.

## Revendications

1. Composition d'hydrogel, comprenant :
un acide polyglutamique (PGA) contenant des groupements maléimide ; et
un polyéthylène glycol (PEG) contenant des groupements thiol terminaux, dans laquelle la composition d'hydrogel a une valeur de pH comprise entre 4,0 et 6,5.

2. Composition d'hydrogel selon la revendication 1, dans laquelle l'acide polyglutamique (PGA) contenant des groupements maléimide a une masse moléculaire comprise entre 10 kDa et 1000 kDa.

3. Composition d'hydrogel selon l'une des revendications 1 et 2, dans laquelle l'acide polyglutamique (PGA) contenant des groupements maléimide a un rapport de greffage compris entre 5 % et 40 %.

4. Composition d'hydrogel selon l'une des revendications 1, 2 et 3, dans laquelle l'acide polyglutamique (PGA) contenant des groupements maléimide a une concentration comprise entre 0,75 % en masse et 10 % en masse dans la composition d'hydrogel.

5. Composition d'hydrogel selon l'une des revendications 1, 2, 3 et 4, dans laquelle l'acide polyglutamique (PGA) contenant des groupements maléimide est exempte de groupements thiol.

6. Composition d'hydrogel selon l'une des revendications 1, 2, 3, 4 et 5, dans laquelle le polyéthylène glycol (PEG) contenant des groupements thiol terminaux a une masse moléculaire comprise entre 2 kDa et 20 kDa.

7. Composition d'hydrogel selon l'une des revendications 1, 2, 3, 4, 5 et 6, dans laquelle le polyéthylène glycol (PEG) contenant des groupements thiol terminaux a une concentration comprise entre 0,75 % en masse et 10 % en masse dans la composition d'hydrogel.

8. Composition d'hydrogel selon l'une des revendications 1, 2, 3, 4, 5, 6 et 7, dans laquelle le polyéthylène glycol (PEG) contenant des groupements thiol terminaux est de type à 4 bras, de type à 8 bras ou en forme de Y.

9. Composition d'hydrogel selon l'une des revendications 1, 2, 3, 4, 5, 6, 7 et 8, dans laquelle le polyéthylène glycol (PEG) contenant des groupements thiol terminaux est exempt de groupements maléimide.

10. Composition d'hydrogel selon l'une des revendications 1, 2, 3, 4, 5, 6, 7, 8 et 9, dans laquelle le polyéthylène glycol (PEG) contenant des groupements thiol terminaux et l'acide polyglutamique (PGA) contenant des groupements maléimide ont un rapport molaire des groupements thiol aux groupements maléimide compris entre 0,2 et 5,0.

11. Composition d'hydrogel selon l'une des revendications 1, 2, 3, 4, 5, 6, 7, 8, 9 et 10, dans laquelle le polyéthylène glycol (PEG) contenant des groupements thiol terminaux et l'acide polyglutamique (PGA) contenant des groupements maléimide ont un rapport molaire des groupements thiol aux groupements maléimide compris entre 1,0 et 1,5.

12. Système de libération de médicament, comprenant :
une composition d'hydrogel selon l'une des revendications 1, 2, 3, 4, 5, 6, 7, 8, 9, 10 et 11 ; et
un principe actif pharmaceutique encapsulé dans la composition d'hydrogel.

13. Système de libération de médicament selon la revendication 12, dans lequel le principe actif pharmaceutique est choisi dans le groupe constitué par les facteurs de croissance, les hormones, les peptides, les protéines, les anticorps, les petites molécules hydrophiles et les petites molécules hydrophobes.

14. Système de libération de médicament selon l'une des revendications 12 et 13, dans lequel le principe actif pharmaceutique est choisi dans le groupe constitué par les anticorps intacts et les fragments d'anticorps.

15. Système de libération de médicament selon l'une des revendications 12, 13 et 14, dans lequel le principe actif pharmaceutique est choisi dans le groupe constitué par les anticorps murins, les anticorps chimériques, les anticorps humanisés et les anticorps humains.

16. Système de libération de médicament selon l'une des revendications 12 et 13, dans lequel le principe actif pharmaceutique est choisi dans le groupe constitué par les agents antinéoplasiques, les antipsychotiques, les analgésiques et les antibiotiques.

17. Système de libération de médicament selon l'une des revendications 12, 13, 14 et 15, dans lequel le principe actif pharmaceutique est en outre associé à un polymère, un métal, des composés chargés ou des particules chargées pour former un complexe de ces derniers.

18. Système de libération de médicament selon la revendication 17, dans lequel le polymère comprend de l'acide polyglutamique (PGA), de l'acide hyaluronique, du chitosane ou du dextrane.

19. Système de libération de médicament selon la revendication 17, dans lequel le métal comprend du zinc, du calcium, du magnésium ou du fer.

20. Système de libération de médicament selon la revendication 17, dans lequel le complexe a une taille comprise entre 10 nm et 100 µm.

21. Système de libération de médicament selon l'une des revendications 12, 17 et 20, dans lequel le principe actif pharmaceutique ou le complexe a une concentration comprise entre 1 mg/mL et 300 mg/mL.
